# EUROPEAN PATENT APPLICATION

(11) **EP 4 257 610 A1**
(43) Date of publication of application: **11.10.2023**
(21) Application number: 22739046.5
(22) Date of filing: 12.01.2022
(51) Int. Cl.: C07K 16/30, C12N 15/13, C12N 15/63, A61K 39/395, A61P 35/00

(54) **ROR1-TARGETING ANTIBODY AND USE THEREOF**

(30) Priority: 12.01.2021 CN 202110037655
(71) Applicant: Bioheng Therapeutics Limited, Grand Cayman KY1-1209 (KY)
(72) Inventor: ZHOU, Yali, Nanjing, Jiangsu 210061 (CN); CHEN, Gong, Nanjing, Jiangsu 210061 (CN); GUO, Tingting, Nanjing, Jiangsu 210061 (CN); JIANG, Xiaoyan, Nanjing, Jiangsu 210061 (CN); REN, Jiangtao, Nanjing, Jiangsu 210061 (CN); HE, Xiaohong, Nanjing, Jiangsu 210061 (CN); WANG, Yanbin, Nanjing, Jiangsu 210061 (CN); HAN, Lu, Nanjing, Jiangsu 210061 (CN); LI, Guokun, Nanjing, Jiangsu 210061 (CN); ZHANG, Jing, Nanjing, Jiangsu 210061 (CN); SUN, Huifang, Nanjing, Jiangsu 210061 (CN)
(74) Representative: Rimini, Rebecca
(86) International application number: PCT/CN2022/071621
(87) International publication number: WO 2022/152168

(57) **Abstract**

Provided are an ROR1-targeting antibody, and a multispecific antibody, a chimeric antigen receptor, an antibody conjugate, a pharmaceutical composition and a kit which comprise same, and the use thereof in the diagnosis/treatment/prevention of diseases associated with ROR1 expression.

## Description

### Technical Field

The present disclosure belongs to the field of immunotherapy. More specifically, the present disclosure relates to a ROR1-targeting antibody and use thereof in the prevention and/or treatment and/or diagnosis of diseases.

### Background Art

Receptor tyrosine kinase-like orphan receptor 1 (ROR1), a member of the receptor tyrosine kinase (RTK) family, is generally highly expressed during embryogenesis and fetal development, but not on normal adult cells. However, the expression of ROR1 has been detected in many hematological and solid malignancies, such as chronic lymphocytic leukaemia (CLL), acute lymphoblastic leukaemia (ALL), mantle cell leukemia, hairy cell leukemia, pancreatic cancer, prostate cancer, colon cancer, bladder cancer, ovarian cancer, testicular cancer, uterine cancer, adrenal cancer, breast cancer, lung cancer, melanoma, neuroblastoma, sarcoma, kidney cancer, etc. Because of this tumor-embryonic expression pattern of ROR1, it has become a target for the treatment of certain cancers and tumors.

Therefore, it is of great value and significance to develop drugs and antibodies against ROR1 target. The present disclosure aims to provide a ROR1-targeting antibody and use thereof in the prevention and/or treatment and/or diagnosis of diseases.

### Summary

In a first aspect, the present disclosure provides a ROR1-targeting antibody, comprising:
(1) CDR-L1 as set forth in SEQ ID NO: 1, CDR-L2 as set forth in SEQ ID NO: 2, CDR-L3 as set forth in SEQ ID NO: 3, CDR-H1 as set forth in SEQ ID NO: 4, CDR-H2 as set forth in SEQ ID NO: 5 and CDR-H3 as set forth in SEQ ID NO: 6;
(2) CDR-L1 as set forth in SEQ ID NO: 1, CDR-L2 as set forth in SEQ ID NO: 2, CDR-L3 as set forth in SEQ ID NO: 7, CDR-H1 as set forth in SEQ ID NO: 4, CDR-H2 as set forth in SEQ ID NO: 8 and CDR-H3 as set forth in SEQ ID NO: 9;
(3) CDR-L1 as set forth in SEQ ID NO: 10, CDR-L2 as set forth in SEQ ID NO: 11, CDR-L3 as set forth in SEQ ID NO: 12, CDR-H1 as set forth in SEQ ID NO: 13, CDR-H2 as set forth in SEQ ID NO: 14 and CDR-H3 as set forth in SEQ ID NO: 15;
(4) CDR-L1 as set forth in SEQ ID NO: 16, CDR-L2 as set forth in SEQ ID NO: 17, CDR-L3 as set forth in SEQ ID NO: 18, CDR-H1 as set forth in SEQ ID NO: 19, CDR-H2 as set forth in SEQ ID NO: 20 and CDR-H3 as set forth in SEQ ID NO: 21; or
(5) CDR-L1 as set forth in SEQ ID NO: 1, CDR-L2 as set forth in SEQ ID NO: 22, CDR-L3 as set forth in SEQ ID NO: 7, CDR-H1 as set forth in SEQ ID NO: 4, CDR-H2 as set forth in SEQ ID NO: 5 and CDR-H3 as set forth in SEQ ID NO: 23.

In an embodiment, the antibody or antigen-binding fragment thereof of the present disclosure comprises a heavy chain variable region and a light chain variable region, wherein the heavy chain variable region has at least 90% identity to an amino acid sequence selected from the group consisting of SEQ ID NOs: 25, 28, 31, 34, 37, 69, 73, 77, 81, 85, 89 and 93, or has modifications of one or more amino acids (for example, at most 1, 2, 3, 4, 5, 6, 7, 8, 9 or 10 amino acids) compared to the amino acid sequence selected from the group consisting of SEQ ID NOs: 25, 28, 31, 34, 37, 69, 73, 77, 81, 85, 89 and 93; the light chain variable region has at least 90% identity to an amino acid sequence selected from the group consisting of SEQ ID NOs: 24, 27, 30, 33, 36, 68, 72, 76, 80, 84, 88 and 92, or has modifications of one or more amino acids (for example, at most 1, 2, 3, 4, 5, 6, 7, 8, 9 or 10 amino acids) compared to the amino acid sequence selected from the group consisting of SEQ ID NOs: 24, 27, 30, 33, 36, 68, 72, 76, 80, 84, 88 and 92. Preferably, the modifications are conservative modifications, such as conservative substitutions, additions and deletions of amino acids. In a preferred embodiment, the antibody or antigen-binding fragment thereof of the present disclosure comprises a heavy chain variable region selected from the group consisting of SEQ ID NOs: 25, 28, 31, 34, 37, 69, 73, 77, 81, 85, 89 and 93 and a light chain variable region selected from the group consisting of SEQ ID NOs: 24, 27, 30, 33, 36, 68, 72, 76, 80, 84, 88 and 92.

In an embodiment, the antibody or antigen-binding fragment thereof of the present disclosure comprises a heavy chain variable region and a light chain variable region selected from:
(a) a heavy chain variable region as set forth in SEQ ID NO: 25 and a light chain variable region as set forth in SEQ ID NO: 24;
(b) a heavy chain variable region as set forth in SEQ ID NO: 28 and a light chain variable region as set forth in SEQ ID NO: 27;
(c) a heavy chain variable region as set forth in SEQ ID NO: 31 and a light chain variable region as set forth in SEQ ID NO: 30;
(d) a heavy chain variable region as set forth in SEQ ID NO: 34 and a light chain variable region as set forth in SEQ ID NO: 33;
(e) a heavy chain variable region as set forth in SEQ ID NO: 37 and a light chain variable region as set forth in SEQ ID NO: 36;
(f) a heavy chain variable region as set forth in SEQ ID NO: 69 and a light chain variable region as set forth in SEQ ID NO: 68;
(g) a heavy chain variable region as set forth in SEQ ID NO: 73 and a light chain variable region as set forth in SEQ ID NO: 72;
(h) a heavy chain variable region as set forth in SEQ ID NO: 77 and a light chain variable region as set forth in SEQ ID NO: 76;
(i) a heavy chain variable region as set forth in SEQ ID NO: 81 and a light chain variable region as set forth in SEQ ID NO: 80;
(j) a heavy chain variable region as set forth in SEQ ID NO: 85 and a light chain variable region as set forth in SEQ ID NO: 84;
(k) a heavy chain variable region as set forth in SEQ ID NO: 89 and a light chain variable region as set forth in SEQ ID NO: 88;
(i) a heavy chain variable region as set forth in SEQ ID NO: 93 and a light chain variable region as set forth in SEQ ID NO: 92.

Optionally, the heavy chain variable region and the light chain variable region have at least 90%, least 91%, least 92%, least 93%, least 94%, least 95%, least 96%, least 97%, least 98%, least 99%, 100% identity to the heavy chain variable region and the light chain variable region of any one of (a)-(l).

Optionally, the heavy chain variable region and the light chain variable region have modifications of one or several amino acids, such as modifications of at most 1, 2, 3, 4, 5, 6, 7, 8, 9 or 10 amino acids compared to the heavy chain variable region and the light chain variable region of any one of (a)-(l); preferably, the modifications are conservative modifications, such as conservative substitutions, additions and deletions of amino acids.

In an embodiment, the antibody or antigen-binding fragment thereof of the present disclosure has at least 90%, at least 91%, at least 92%, at least 93%, at least 94%, at least 95%, at least 96%, at least 97%, at least 98%, at least 99%, 100% identity to an amino acid sequence selected from the group consisting of SEQ ID NOs: 26, 29, 32, 35, 38, 70, 74, 78, 82, 86, 90 and 94, or has modifications of one or several amino acids, such as modifications of at most 1, 2, 3, 4, 5, 6, 7, 8, 9 or 10 amino acids compared to the amino acid sequence selected from the group consisting of SEQ ID NOs: 26, 29, 32, 35, 38, 70, 74, 78, 82, 86, 90 and 94. Preferably, the modifications are conservative modifications, such as conservative substitutions, additions and deletions of amino acids. Preferably, the antibody or antigen-binding fragment thereof of the present disclosure has an amino acid sequence selected from the group consisting of SEQ ID NOs: 26, 29, 32, 35, 38, 70, 74, 78, 82, 86, 90 and 94.

In an embodiment, the antibody or antigen-binding fragment thereof of the present disclosure is a murine antibody, a chimeric antibody, a humanized antibody or a human antibody, preferably a humanized antibody.

The present disclosure further provides a nucleic acid molecule encoding the antibody or antigen-binding fragment thereof as described above. Accordingly, in an embodiment, the nucleic acid molecule encoding the antibody or antigen-binding fragment thereof has at least 90%, at least 91%, at least 92%, at least 93%, at least 94%, at least 95%, at least 96%, at least 97%, at least 98%, at least 99%, 100% sequence identity to a nucleotide sequence selected from the group consisting of SEQ ID NOs: 39-43, 71, 75, 78, 83, 87, 91 and 95, and the encoded antibody or antigen-binding fragment thereof is capable of specifically binding to ROR1 antigen. Preferably, the nucleic acid molecule encoding the antibody or antigen-binding fragment thereof is selected from SEQ ID NOs: 39-43, 71, 75, 78, 83, 87, 91 and 95.

In another aspect, the present disclosure further provides a multispecific antibody (preferably bispecific antibody or trispecific antibody), which comprises the anti-ROR1 antibody or antigen-binding fragment thereof as described above, and one or more second antibodies or antigen-binding portions thereof that specifically bind to antigens different from ROR1.

In an embodiment, the second antibody/antibodies or antigen-binding portion thereof may be in the form of any antibody or antibody fragment, such as full-length antibody, Fab, Fab', (Fab')₂, Fv, scFv, scFv-scFv, minibody, diabody or sdAb.

The present disclosure further provides a vector comprising a nucleic acid molecule encoding the anti-ROR1 antibody or antigen-binding fragment thereof or the multispecific antibody as described above, and a host cell expressing the anti-ROR1 antibody or antigen-binding fragment thereof or the multispecific antibody.

In another aspect, the present disclosure further provides a chimeric antigen receptor, which comprises the anti-ROR1 antibody or antigen-binding fragment thereof of the present disclosure, a transmembrane domain and an intracellular signaling domain. Preferably, the chimeric antigen receptor further comprises one or more co-stimulatory domains.

The present disclosure further provides a nucleic acid molecule encoding a chimeric antigen receptor targeting ROR1 as defined above, and a vector comprising the nucleic acid molecule.

The present disclosure further provides a cell, preferably an immune cell, such as a T cell, an NK cell, an NKT cell, a macrophage, a dendritic cell, comprising a chimeric antigen receptor targeting ROR1 as defined above. In a preferred embodiment, the engineered immune cells further comprise a second chimeric antigen receptor targeting a tumor antigen different from ROR1.

In another aspect, the present disclosure further provides an antibody conjugate comprising the anti-ROR1 antibody or antigen-binding fragment thereof defined in the present disclosure and a second functional structure, wherein the second functional structure is selected from the group consisting of an Fc, a radioisotope, a structure moiety for extending half-life, a detectable marker and a drug.

In an embodiment, the structure moiety for extending half-life is selected from the group consisting of an albumin-binding structure, a transferrin-binding structure, a polyethylene glycol molecule, a recombinant polyethylene glycol molecule, a human serum albumin, a fragment of human serum albumin, and a polypeptide (including an antibody) binding to human serum albumin. In an embodiment, the detectable marker is selected from the group consisting of a fluorophore, a chemiluminescent compound, a bioluminescent compound, an enzyme, an antibiotic resistance gene, and a contrast agent. In an embodiment, the drug is selected from the group consisting of a cytotoxin and an immunomodulator.

In another aspect, the present disclosure further provides a detection kit comprising the anti-ROR1 antibody or antigen-binding fragment thereof, the multispecific antibody, the antibody conjugate, the chimeric antigen receptor or the engineered immune cell described in the present disclosure.

In another aspect, the present disclosure further provides a pharmaceutical composition comprising the anti-ROR1 antibody or antigen-binding fragment thereof, the chimeric antigen receptor, the multispecific antibody, the engineered cell or the antibody conjugate described in the present disclosure, and one or more pharmaceutically acceptable excipients.

In another aspect, the present disclosure further provides a method for treating and/or preventing and/or diagnosing diseases associated with ROR1 expression, comprising administering to a subject the anti-ROR1 antibody or antigen-binding fragment thereof, the chimeric antigen receptor, the multispecific antibody, the antibody conjugate, the engineered immune cell or the pharmaceutical composition as described above.

### Brief Description of Drawings

FIG. 1 shows the scFv expression level of CAR-T cells expressing anti-ROR1 murine scFv.
FIG. 2 shows the killing effect of CAR-T cells expressing anti-ROR1 murine scFv on target cells at different effector-to-target ratios.
FIG. 3 shows the IFN-γ release level after co-culture of CAR-T cells expressing anti-ROR1 murine scFv with various target cells and non-target cells.
FIG. 4 shows scFv expression level of CAR-T cells expressing anti-ROR1 humanized scFv.
FIG. 5 shows the killing effect of CAR-T cells expressing anti-ROR1 humanized scFv on target cell Jeko-1-luci (A) and non-target cell K562-luci (B) at different effector-to-target ratios.
FIG. 6 shows the degranulation effect of CAR-T cells expressing anti-ROR1 humanized scFv on target cells MDA-MB-231, Jeko-1, A549 and non-target cell K562.
FIG. 7 shows the IL2 (A) and IFN-γ (B) release level after co-culture of CAR-T cells expressing anti-ROR1 humanized scFv with target cells MDA-MB-231, Jeko-1, A549 and non-target cell K562.

### Detailed Description of Embodiments

Unless otherwise specified, all scientific and technical terms used herein have the same meaning as commonly understood by one of ordinary skill in the art to which this disclosure belongs.

### Anti-ROR1 antibody or antigen-binding fragment thereof

As used herein, the term "antibody" has the broadest meaning understood by those skilled in the art and includes monoclonal antibodies (including whole antibodies), polyclonal antibodies, multivalent antibodies, multispecific antibodies (e.g., bispecific antibodies), and antibody fragments or synthetic polypeptides carrying one or more CDR sequences capable of exhibiting the desired biological activity. The antibodies of the present disclosure may be of any class (e.g., IgG, IgE, IgM, IgD, IgA, etc.) or subclass (e.g., IgG1, IgG2, IgG2a, IgG3, IgG4, IgA1, IgA2, etc.).

As used herein, the term "antigen-binding fragment" or "antibody fragment" refers to one or more fragments of an antibody that retain the ability to specifically bind an antigen. It has been shown that the antigen-binding function of antibodies can be performed by fragments of full-length antibodies. Examples of antibody fragments of the present disclosure include, but are not limited to: Fab, Fab', F(ab')2, Fd fragment, Fd', Fv fragment, single-chain antibody (scFv), disulfide-linked Fv (sdFv), antibody heavy chain variable region (VH) or light chain variable region (VL), linear antibody, "diabody" with two antigen binding sites, single domain antibody, nanobody, a natural ligand for the antigen or a functional fragment thereof. Thus, unless the context clearly dictates otherwise, an "antibody" of the present invention encompasses antibody fragments or antigen-binding fragments as defined above. Thus, in a preferred embodiment, the antibody of the invention is selected from the group consisting of IgG, Fab, Fab', F(ab')₂, Fd, Fd', Fv, scFv, sdFv, linear antibody and diabody.

Typically, whole antibodies comprise two heavy chains and two light chains disulfide-bonded together, each light chain being disulfide-bonded to a respective heavy chain, to form a "Y" configuration. Each heavy chain comprises a heavy chain variable region (VH) and a heavy chain constant region, wherein the heavy chain variable region comprises three complementarity determining regions (CDRs): CDR-H1, CDR-H2 and CDR-H3, and the heavy chain constant region comprises three constant domains: CH1, CH2 and CH3. Each light chain comprises a light chain variable region (VL) and a light chain constant region, wherein the light chain variable region comprises three CDRs: CDR-L1, CDR-L2 and CDR-L3, and the light chain constant region comprises a constant domain CL. In the heavy/light chain variable regions, the CDRs are separated by more conserved framework regions (FRs). The heavy/light chain variable regions recognize and bind antigen, while the constant regions mediate the binding of the antibody to host tissues or factors, including various cells of the immune system (e.g., effector cells) and the first component of the classical complement system.

The precise amino acid sequence boundaries for a given CDR or FR can be readily determined using a number of numbering schemes well known in the art, including: Kabat et al. (1991), "Sequences of Proteins of Immunological Interest," 5th Edition, Public Health Service, National Institutes of Health, Bethesda, MD ("Kabat" numbering scheme); Al-Lazikani et al., (1997) JMB 273, 927-948 ("Chothia" numbering scheme); MacCallum et al., J. Mol. Biol 262:732-745 (1996), "Antibody-antigen interactions: Contact analysis and binding sitetopography," J. Mol. Biol. 262, 732-745" ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," JMol Biol, 2001 Jun 8; 309(3):657-70 ("Aho" numbering scheme); and Martin et al., "Modeling antibody hypervariable loops: a combined algorithm," PNAS, 1989, 86(23):9268-9272 ("AbM" numbering scheme).

The boundaries of a given CDR or FR may vary depending on the scheme used for identification. For example, the Kabat scheme is based on structural alignment, while the Chothia scheme is based on structural information. Both the Kabat and Chothia numbering schemes are based on the sequence length of the most common antibody regions where insertions are provided by caret letters (e.g., "30a") and deletions occur in some antibodies. These two schemes place certain insertions and deletions (indels) at different positions, resulting in different numbering. The Contact scheme is based on the analysis of complex crystal structures and is similar in many respects to the Chothia numbering scheme. The AbM scheme is a compromise between the Kabat and Chothia definitions and is based on the scheme used by the AbM antibody modeling software of Oxford Molecular.

Thus, unless otherwise specified, a "CDR" of a given antibody or region thereof (e.g., variable region thereof) is understood to encompass the CDRs defined by any of the above schemes or other known schemes. For example, where it is specified that a particular CDR (e.g., CDR3) comprises a given amino acid sequence, it is understood that such a CDR may also have the sequence of the corresponding CDR (e.g., CDR3) as defined by any of the above schemes or other known schemes. Likewise, unless otherwise specified, FRs for a given antibody or region thereof (e.g., variable region thereof) are understood to encompass FRs as defined by any of the above schemes or other known schemes. Unless otherwise specified, the numbering scheme used herein to define the boundaries of CDRs and FRs adopts the Chothia scheme.

"Single-chain antibody" and "scFv" are used interchangeably herein and refer to an antibody formed by linking the heavy chain variable region (VH) and the light chain variable region (VL) of an antibody through a linker. The optimal length and/or amino acid composition of the linker can be selected. The length of the linker may significantly affect the folding and interaction of the variable domain of scFv. In fact, if shorter linkers (e.g., with between 5-10 amino acids) are used, intrachain folding may be prevented. For selection of linker size and composition, see, e.g., Hollinger et al., 1993 Proc Natl Acad. Sci. U.S.A. 90:6444-6448; U.S. Patent Application Publication Nos. 2005/0100543, 2005/0175606, 2007/0014794 and PCT Publication Nos. WO2006/020258 and WO2007/024715, the entire contents of which are incorporated herein by reference. Commonly used linkers are, for example, GSTSGSGKPGSGEGSTKG (SEQ ID NO: 66), GGGGSGGGGSGGGGS (SEQ ID NO: 67). A scFv may comprise VH and VL linked in any order, e.g. VH-linker-VL or VL-linker-VH.

In an embodiment, the antibody or antigen-binding fragment thereof of the present disclosure is a murine antibody, a chimeric antibody, a humanized antibody or a human antibody, preferably a humanized antibody.

As used herein, the term "chimeric antibody" refers to an antibody in which a portion of the amino acid sequence of each of the heavy and light chains is homologous to the corresponding sequence in an antibody from a particular species or belonging to a particular class, while the remaining segments of the chain are homologous to the corresponding sequences in another species or belonging to another class. Typically, the variable regions of both the light and heavy chains are derived from the variable regions of antibodies from one species, while the constant regions are homologous to antibody sequences from another species. A distinct advantage of this chimeric format is that the variable regions can be conveniently produced from presently known sources using readily available B cells or hybridomas from non-human hosts, and the constant regions combined therewith may be from, for example, human cells. The variable region has the advantage of being easy to prepare, and the specificity is not affected by the source, and since the constant region is of human origin, the antibody will be less likely to elicit an immune response in humans when injected than if the constant region is of non-human origin.

As used herein, a "humanized" antibody refers to an antibody in which all or substantially all CDR amino acid residues are derived from non-human CDRs and all or substantially all FR amino acid residues are derived from human FRs. "Humanized forms" of non-human antibodies refer to variants of such non-human antibodies that have been humanized to generally reduce immunogenicity in humans, while retaining the specificity and affinity of the parent non-human antibody. In some embodiments, some FR residues in a humanized antibody are substituted with corresponding residues from a non-human antibody (e.g., an antibody from which the CDR residues are derived), e.g., to restore or improve antibody specificity or affinity.

Humanized antibodies and methods for their preparation are well known to those skilled in the art, see e.g. Almagro and Fransson, Front. Biosci. 13:1619-1633 (2008). Human framework regions that can be used for humanization include, but are not limited to: framework regions selected using a "best fit" approach; framework regions derived from the consensus sequences of human antibodies of a particular subgroup of light or heavy chain variable regions; human mature (somatically mutated) framework regions or human germline framework regions; and framework regions obtained from screening FR libraries.

As used herein, the term "human antibody" includes antibodies having variable and constant regions derived from human germline immunoglobulin sequences. The human antibody of the present disclosure may comprise amino acid residues not encoded by human germline immunoglobulin sequences (e.g., mutations introduced by random or site-directed mutagenesis in vitro or by somatic mutation in vivo).

In an embodiment, the present disclosure provides a ROR1-targeting antibody or an antigen-binding fragment thereof, comprising:
(1) CDR-L1 as set forth in SEQ ID NO: 1, CDR-L2 as set forth in SEQ ID NO: 2, CDR-L3 as set forth in SEQ ID NO: 3, CDR-H1 as set forth in SEQ ID NO: 4, CDR-H2 as set forth in SEQ ID NO: 5 and CDR-H3 as set forth in SEQ ID NO: 6;
(2) CDR-L1 as set forth in SEQ ID NO: 1, CDR-L2 as set forth in SEQ ID NO: 2, CDR-L3 as set forth in SEQ ID NO: 7, CDR-H1 as set forth in SEQ ID NO: 4, CDR-H2 as set forth in SEQ ID NO: 8 and CDR-H3 as set forth in SEQ ID NO: 9;
(3) CDR-L1 as set forth in SEQ ID NO: 10, CDR-L2 as set forth in SEQ ID NO: 11, CDR-L3 as set forth in SEQ ID NO: 12, CDR-H1 as set forth in SEQ ID NO: 13, CDR-H2 as set forth in SEQ ID NO: 14 and CDR-H3 as set forth in SEQ ID NO: 15;
(4) CDR-L1 as set forth in SEQ ID NO: 16, CDR-L2 as set forth in SEQ ID NO: 17, CDR-L3 as set forth in SEQ ID NO: 18, CDR-H1 as set forth in SEQ ID NO: 19, CDR-H2 as set forth in SEQ ID NO: 20 and CDR-H3 as set forth in SEQ ID NO: 21; or
(5) CDR-L1 as set forth in SEQ ID NO: 1, CDR-L2 as set forth in SEQ ID NO: 22, CDR-L3 as set forth in SEQ ID NO: 7, CDR-H1 as set forth in SEQ ID NO: 4, CDR-H2 as set forth in SEQ ID NO: 5 and CDR-H3 as set forth in SEQ ID NO: 23.

In an embodiment, the antibody or antigen-binding fragment thereof of the present disclosure comprises a heavy chain variable region and a light chain variable region, wherein the heavy chain variable region has at least 90%, at least 91%, at least 92%, at least 93%, at least 94%, at least 95%, at least 96%, at least 97%, at least 98%, at least 99%, or 100% identity to an amino acid sequence selected from the group consisting of SEQ ID NOs: 25, 28, 31, 34, 37, 69, 73, 77, 81, 85, 89 and 93, or has modifications of one or more amino acids (for example, at most 1, 2, 3, 4, 5, 6, 7, 8, 9 or 10 amino acids) compared to the amino acid sequence selected from the group consisting of SEQ ID NOs: 25, 28, 31, 34, 37, 69, 73, 77, 81, 85, 89 and 93; the light chain variable region has at least 90%, at least 91%, at least 92%, at least 93%, at least 94%, at least 95%, at least 96%, at least 97%, at least 98%, at least 99%, or 100% identity to an amino acid sequence selected from the group consisting of SEQ ID NOs: 24, 27, 30, 33, 36, 68, 72, 76, 80, 84, 88 and 92, or has modifications of one or more amino acids (for example, at most 1, 2, 3, 4, 5, 6, 7, 8, 9 or 10 amino acids) compared to the amino acid sequence selected from the group consisting of SEQ ID NOs: 24, 27, 30, 33, 36, 68, 72, 76, 80, 84, 88 and 92. Preferably, the modifications are conservative modifications, such as conservative substitutions, additions and deletions of amino acids. In a preferred embodiment, the antibody or antigen-binding fragment thereof of the present disclosure comprises a heavy chain variable region selected from the group consisting of SEQ ID NOs: 25, 28, 31, 34, 37, 69, 73, 77, 81, 85, 89 and 93 and a light chain variable region selected from the group consisting of SEQ ID NOs: 24, 27, 30, 33, 36, 68, 72, 76, 80, 84, 88 and 92. In an embodiment, the antibody or antigen-binding fragment thereof of the present disclosure comprises a heavy chain variable region and a light chain variable region selected from the group consisting of:
(a) a heavy chain variable region as set forth in SEQ ID NO: 25 and a light chain variable region as set forth in SEQ ID NO: 24;
(b) a heavy chain variable region as set forth in SEQ ID NO: 28 and a light chain variable region as set forth in SEQ ID NO: 27;
(c) a heavy chain variable region as set forth in SEQ ID NO: 31 and a light chain variable region as set forth in SEQ ID NO: 30;
(d) a heavy chain variable region as set forth in SEQ ID NO: 34 and a light chain variable region as set forth in SEQ ID NO: 33;
(e) a heavy chain variable region as set forth in SEQ ID NO: 37 and a light chain variable region as set forth in SEQ ID NO: 36;
(f) a heavy chain variable region as set forth in SEQ ID NO: 69 and a light chain variable region as set forth in SEQ ID NO: 68;
(g) a heavy chain variable region as set forth in SEQ ID NO: 73 and a light chain variable region as set forth in SEQ ID NO: 72;
(h) a heavy chain variable region as set forth in SEQ ID NO: 77 and a light chain variable region as set forth in SEQ ID NO: 76;
(i) a heavy chain variable region as set forth in SEQ ID NO: 81 and a light chain variable region as set forth in SEQ ID NO: 80;
(j) a heavy chain variable region as set forth in SEQ ID NO: 85 and a light chain variable region as set forth in SEQ ID NO: 84;
(k) a heavy chain variable region as set forth in SEQ ID NO: 89 and a light chain variable region as set forth in SEQ ID NO: 88;
(i) a heavy chain variable region as set forth in SEQ ID NO: 93 and a light chain variable region as set forth in SEQ ID NO: 92.

Optionally, the heavy chain variable region and the light chain variable region have at least 90%, at least 91%, at least 92%, at least 93%, at least 94%, at least 95%, at least 96%, at least 97%, at least 98%, at least 99%, or 100% identity to the heavy chain variable region and light chain variable region of any one of (a)-(l).

Optionally, the heavy chain variable region and the light chain variable region have modifications of one or several amino acids, such as modifications of at most 1, 2, 3, 4, 5, 6, 7, 8, 9 or 10 amino acids compared to the heavy chain variable region and the light chain variable region of any one of (a)-(l); preferably, the modifications are conservative modifications, such as conservative substitutions, additions and deletions of amino acids.

In an embodiment, the antibody or antigen-binding fragment thereof of the present disclosure has at least 90%, at least 91%, at least 92%, at least 93%, at least 94%, at least 95%, at least 96%, at least 97%, at least 98%, at least 99%, or 100% identity to an amino acid sequence selected from the group consisting of SEQ ID NOs: 26, 29, 32, 35, 38, 70, 74, 78, 82, 86, 90 and 94, or has modifications of one or several amino acids, such as modifications of at most 1, 2, 3, 4, 5, 6, 7, 8, 9 or 10 amino acids compared to the amino acid sequence selected from the group consisting of SEQ ID NOs: 26, 29, 32, 35, 38, 70, 74, 78, 82, 86, 90 and 94. Preferably, the modifications are conservative modifications, such as conservative substitutions, additions and deletions of amino acids. Preferably, the antibody or antigen-binding fragment thereof of the present disclosure has an amino acid sequence selected from the group consisting of SEQ ID NOs: 26, 29, 32, 35, 38, 70, 74, 78, 82, 86, 90 and 94.

As used herein, the term "conservative modification" refers to an amino acid modification that does not significantly affect or alter the binding characteristics of an antibody or antibody fragment comprising the amino acid sequence. These conservative modifications include conservative substitutions, additions and deletions of amino acids. Modifications can be introduced into the antibodies of the present disclosure by standard techniques known in the art, such as site-directed mutagenesis and PCR-mediated mutagenesis. A conservative substitution of amino acid is one in which an amino acid residue is replaced by an amino acid residue with a similar side chain. Families of amino acid residues with similar side chains have been defined in the art and include those with basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), non-polar side chain (e.g., alanine, valine, leucine, isoleucine, proline, phenylalanine, methionine, tryptophan), beta-branched side chain (e.g., threonine, valine, isoleucine) and aromatic side chain (e.g., tyrosine, phenylalanine, tryptophan, histidine). Conservative modifications can be selected, for example, on the basis of similarity in polarity, charge, solubility, hydrophobicity, hydrophilicity, and/or the amphipathic nature of the residues involved.

As used herein, the term sequence "identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art know that several algorithms can be used to determine sequence identity, such as Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

In an aspect, the present disclosure further provides a multispecific antibody (preferably a bispecific antibody or a trispecific antibody) comprising the anti-ROR1 antibody or antigen-binding fragment thereof as described above, and one or more second antibodies that specifically bind to antigen(s) different from ROR1.

As used herein, the term "multispecific" means that the antigen binding protein has polyepitopic specificity (i.e., is capable of specifically binding two, three or more different epitopes on one biomolecule or is capable of specifically binding epitopes on two, three or more different biomolecules). As used herein, the term "bispecific" means that an antigen binding protein has two different antigen binding specificities.

In an embodiment, the second antibody may be in the form of any antibody or antibody fragment, such as full-length antibody, Fab, Fab', (Fab')₂, Fv, scFv, scFv-scFv, minibody, diabody or sdAb.

Thus, in an embodiment, the second antibody targets an antigen selected from the group consisting of: BCMA, CD4, CD5, CD7, CD8, CD14, CD15, CD19, CD20, CD21, CD22, CD23, CD25, CD33, CD37, CD38, CD40, CD40L, CD46, CD52, CD54, CD80, CD126, CD138, B7, MUC-1, Ia, HM1.24, HLA-DR, tenascin, angiogenic factor, VEGF, PIGF, ED-B fibronectin, oncogene, oncogene product, CD66a-d, necrosis antigen, Ii, IL-2, T101, TAC, IL-6, DR4, DR5, tEGFR, Her2, L1-CAM, mesothelin, CEA, hepatitis B surface antigen, antifolate receptor, CD24, CD30, CD44, EGFR, EGP-2, EGP-4, EPHa2, ErbB2, ErbB3, ErbB4, ErbB dimer, EGFR vIII, FBP, FCRL5, FCRH5, fetal acetylcholine receptor, GD2, GD3, GPRC5D, HMW-MAA, IL-22R-α, IL-13R-α2, kdr, κ light chain, Lewis Y, L1-CAM, MAGE-A1, MAGE-A3, MAGE-A6, PRAME, survivin, EGP2, EGP40, TAG72, B7-H6, IL-13Ra2, CA9, CD171, G250/CAIX, HLA-A1, HLA-A2, NY-ESO-1, PSCA, folate receptor-a, CD44v6, CD44v7/8, avb6 integrin, 8H9, NCAM, VEGF receptor, 5T4, fetal AchR, NKG2D ligand, dual antigens, antigens associated with common tags, cancer-testis antigen, MUC1, MUC16, NY-ESO-1, MART-1, gplOO, carcinoembryonic antigen, VEGF-R2, CEA, prostate specific antigen, PSMA, Her2/neu, estrogen receptor, progesterone receptor, ephrin B2, CD123, c-Met, GD-2, OGD2, CE7, WT-1, cyclin A2, CCL-1, hTERT, MDM2, CYP1B, WT1, survivin, AFP, p53, cyclin (D1), CS-1, BAFF-R, TACI, CD56, TIM-3, CD123, L1-cell adhesion molecule, cell cyclins (e.g., cyclin A1 (CCNA1)) and/or pathogen-specific antigens, biotinylated molecules, molecules expressed by HIV, HCV, HBV and/or other pathogens; and/or neo-epitopes or neoantigens.

### Nucleic acid, vector, host cell

In another aspect, the present disclosure relates to a nucleic acid molecule encoding the anti-ROR1 antibody or multispecific antibody of the present disclosure. The nucleic acid of the present disclosure may be RNA, DNA or cDNA. According to an embodiment of the present disclosure, the nucleic acid of the present disclosure is a substantially isolated nucleic acid.

In an embodiment, the nucleic acid molecule encoding the anti-ROR1 antibody has at least 90% at least 91%, at least 92% at least 93%, at least 94%, at least 95%, at least 96%, at least 97%, at least 98%, at least 99%, 100% sequence identity to a nucleotide sequence selected from the group consisting of SEQ ID NOs: 39-43, 71, 75, 78, 83, 87, 91 and 95, and the anti-ROR1 antibody encoded thereby specifically binds to ROR1(i.e. almost not binding to non-target antigens)). Preferably, the nucleic acid molecule encoding the anti-ROR1 antibody is selected from SEQ ID NOs: 39-43, 71, 75, 78, 83, 87, 91 and 95.

The nucleic acid of the present disclosure may also be in the form of a vector, may be present in a vector and/or may be part of a vector, such as a plasmid, cosmid or YAC. The vector may especially be an expression vector, i.e., a vector providing for expression of the anti-ROR1 antibody in vitro and/or in vivo (i.e., in a suitable host cell, host organism and/or expression system). The expression vector typically comprises at least one nucleic acid molecule of the present disclosure operably linked to one or more suitable expression regulatory elements (e.g., promoter, enhancer, terminator, etc.). Selection of such regulatory elements and their sequences for expression in a particular host is well known to those skilled in the art. Specific examples of regulatory elements and other elements useful or necessary for expression of the anti-ROR1 antibody of the present disclosure include, but are not limited to, promoter, enhancer, terminator, integrator, selectable marker, leader sequence, reporter gene.

In another aspect, the present disclosure further provides a host cell expressing the anti-ROR1 antibody, the multispecific antibody of the present disclosure and/or a host cell comprising the nucleic acid or vector of the present disclosure. Preferred host cells of the present disclosure are bacterial cells, fungal cells or mammalian cells.

Suitable bacterial cells include cells of Gram-negative bacterial strains (e.g., *Escherichia coli* strains, *Proteus* strains, and *Pseudomonas* strains) and Gram-positive bacterial strains (e.g., *Bacillus* strains, *Streptomyces* strains, *Staphylococcus* strains and *Lactococcus* strains).

Suitable fungal cells include cells of species of *Trichoderma*, *Neurospora*, and *Aspergillus*; or cells of species of *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*) and *Hansenula.*

Suitable mammalian cells include, for example, HEK293 cells, CHO cells, BHK cells, HeLa cells, COS cells, and the like.

However, amphibian cells, insect cells, plant cells, and any other cells known in the art for expressing heterologous proteins can also be used in the present disclosure.

### Recombinant receptor

In another aspect, the present disclosure further provides a recombinant receptor, such as a T cell fusion protein, a T cell antigen coupler, a recombinant TCR receptor or a chimeric antigen receptor, comprising the anti-ROR1 antibody as described above. Preferably, the present disclosure further provides a chimeric antigen receptor comprising the anti-ROR1 antibody as described above.

As used herein, the term "T cell fusion protein" or "TFP" refers to a recombinant polypeptide derived from components of a TCR, usually composed of a TCR subunit and an antibody linked thereto, and expressed on the cell surface. The TCR subunit includes at least part of the TCR extracellular domain, the transmembrane domain, and the TCR intracellular signaling domain.

As used herein, the term "T cell antigen coupler" or "TAC" includes three functional domains: 1) tumor targeting domain, including single chain antibody (such as the anti-ROR1 antibody of the present disclosure), designed ankyrin repeat protein (DARPin) or other targeting groups; 2) extracellular domain comprising CD3-binding single-chain antibody, so that TAC receptors and TCR receptors are close to each other; 3) transmembrane domain and the intracellular domain of the CD4 co-receptor, wherein the intracellular domain is linked to the protein kinase LCK, to catalyze the phosphorylation of the immunoreceptor tyrosine activation motif (ITAM) of the TCR complex as an initial step in T cell activation.

As used herein, the term "T cell receptor" or "TCR" is a characteristic marker on the surface of T cells that binds to CD3 in a non-covalent manner to form a complex. Antigen presenting cells present antigenic peptides to T cells through major histocompatibility complex molecules (MHC) and bind to TCR complexes to induce a series of intracellular signaling. TCR is composed of six peptide chains that form heterodimers, which are generally divided into αβ type and γδ type. Each peptide chain includes a constant region and a variable region, where the variable region is responsible for binding specific antigen and MHC molecules.

As used herein, the term "chimeric antigen receptor" or "CAR" refers to an artificially constructed hybrid polypeptide that generally includes a ligand-binding domain (e.g., an antigen-binding portion of an antibody), a transmembrane domain, an optional co-stimulatory domain, and an intracellular signaling domain, which domains being connected by linkers. CARs are able to redirect the specificity and reactivity of T cells and other immune cells to a selected target in a non-MHC-restricted manner through the antigen-binding properties of antibodies.

In an embodiment, the present disclosure provides a chimeric antigen receptor comprising the anti-ROR1 antibody or antigen-binding fragment thereof or the multispecific antibody comprising the anti-ROR1 antibody as described above, a transmembrane domain and an intracellular signaling domain.

As used herein, the term "transmembrane domain" refers to a polypeptide structure that enables expression of a chimeric antigen receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric antigen receptor binds to the target antigen. The transmembrane domains particularly suitable for use in the present disclosure may be derived from, for example, a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, CD154, and functional fragments thereof. Alternatively, the transmembrane domain may be synthesized and may mainly comprise hydrophobic residues such as leucine and valine. Preferably, the transmembrane domain is derived from CD8α chain or CD28, and has at least 70%, preferably at least 80%, more preferably at least 90%, at least 95%, at least 97% or at least 99% or 100% sequence identity to the amino acid sequence as set forth in SEQ ID NO: 44 or 46, or the encoding sequence thereof has at least 70%, preferably at least 80%, more preferably at least 90%, at least 95%, at least 97% or at least 99% or 100% sequence identity to a nucleic acid molecule as set forth in SEQ ID NO: 45 or 47.

As used herein, the term "intracellular signaling domain" refers to a protein portion that transduces an effector function signal and guides a cell to perform a specified function. In an embodiment, the intracellular signaling domain comprised in the chimeric antigen receptor of the present disclosure may be intracellular sequences of a T cell receptor and a co-receptor, upon binding of antigen receptor, which act together to initiate signaling, as well as any derivative or variant of these sequences and any synthetic sequence having the same or similar function. The intracellular signaling domain may comprise many immunoreceptor tyrosine-based activation motifs (ITAM). Non-limiting examples of intracellular signaling domain of the present disclosure include, but are not limited to, intracellular regions of FcRγ, FcRβ, CD3γ, CD3δ, CD3ε, CD3ζ, CD22, CD79a, CD79b, and CD66d. In a preferred embodiment, the signaling domain of the CAR of the present disclosure may comprise a CD3ζ intracellular region, which has at least 70%, preferably at least 80%, more preferably at least 90%, at least 95%, at least 97%, or at least 99% or 100% sequence identity to the amino acid sequence as set forth in SEQ ID NO: 52 or 54, or the encoding sequence thereof has at least 70%, preferably at least 80%, more preferably at least 90%, at least 95%, at least 97%, or at least 99% or 100% sequence identity to a nucleic acid molecule as set forth in SEQ ID NO: 53 or 55.

In an embodiment, the chimeric antigen receptors of the present disclosure may further comprise a hinge region located between the antibody and the transmembrane domain. As used herein, the term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region comprises a hinge region portion of CD8α, CD28, an Fc γ RIII α receptor, IgG4, or IgG1, more preferably a hinge from CD8α, CD28 or IgG4, and has at least 70%, preferably at least 80%, more preferably at least 90%, at least 95%, at least 97% or at least 99% or 100% sequence identity to the amino acid sequence as set forth in SEQ ID NO: 60, 62 or 64, or the encoding sequence thereof has at least 70%, preferably at least 80%, more preferably at least 90%, at least 95%, at least 97% or at least 99% or 100% sequence identity to a nucleic acid molecule as set forth in SEQ ID NO: 61, 63 or 65.

In an embodiment, the chimeric antigen receptor may also comprise one or more co-stimulatory domains. The co-stimulatory domain may be an intracellular functional signaling domain from a co-stimulatory molecule, which comprises the entire intracellular portion of the co-stimulatory molecule, or a functional fragment thereof. A "co-stimulatory molecule" refers to a cognate binding partner that specifically binds to a co-stimulatory ligand on a T cell, thereby mediating a co-stimulatory response (e.g., proliferation) of the T cell. co-stimulatory molecules include, but are not limited to, MHC class 1 molecules, BTLA, and Toll ligand receptors. Non-limiting examples of co-stimulatory domains of the present disclosure include, but are not limited to, co-stimulatory signaling domains derived from the following proteins: TLR1, TLR2, TLR3, TLR4, TLR5, TLR6, TLR7, TLR8, TLR9, TLR10, CARD11, CD2, ROR1, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, LAT, NKG2C, SLP76, PD-1, LIGHT, TRIM, and ZAP70. Preferably, the co-stimulatory domain of the CAR of the present disclosure is from 4-1BB, CD28 or 4-1BB+CD28. In an embodiment, the 4-1BB co-stimulatory domain has at least 70%, preferably at least 80%, more preferably at least 90%, at least 95%, at least 97% or at least 99% or 100% sequence identity to the amino acid sequence as set forth in SEQ ID NO: 50, or the coding sequence thereof has at least 70%, preferably at least 80%, more preferably at least 90%, at least 95%, at least 97% or at least 99% or 100% sequence identity to the nucleic acid molecule as set forth in SEQ ID NO: 51. In an embodiment, the CD28 co-stimulatory domain has at least 70%, preferably at least 80%, more preferably at least 90%, at least 95%, at least 97% or at least 99% or 100% sequence identity to the amino acid sequence as set forth in SEQ ID NO: 48, or the coding sequence thereof has at least 70%, preferably at least 80%, more preferably at least 90%, at least 95%, at least 97% or at least 99% or 100% sequence identity to the nucleic acid molecule as set forth in SEQ ID NO: 49.

In an embodiment, the CAR of the present disclosure may further comprise a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. At the end of the signal peptide, there is usually an amino acid segment capable of being recognized and cleaved by signal peptidase. The signal peptidase may cleave during or after translocation, so as to generate free signal peptide and mature protein. Then, the free signal peptide is digested by a specific protease. Signal peptides that may be used in the present disclosure are well known to those skilled in the art, for example, signal peptides derived from B2M, CD8α, IgG1, GM-CSFRα, and the like. In an embodiment, the signal peptide that can be used in the present disclosure is from B2M or CD8α, and has at least 70%, preferably at least 80%, more preferably at least 90%, at least 95%, at least 97% or at least 99% or 100% sequence identity to the amino acid sequence as set forth in SEQ ID NO: 56 or 58, or the coding sequence thereof has at least 70%, preferably at least 80%, more preferably at least 90%, at least 95%, at least 97% or at least 99% or 100% sequence identity to the nucleic acid molecule as set forth in SEQ ID NO: 57 or 59.

In an embodiment, the CAR comprises the anti-ROR1 antibody or antigen-binding fragment thereof or the multispecific antibody comprising the anti-ROR1 antibody as provided herein, a CD8α or CD28 transmembrane region, a CD28 and/or 4-1BB co-stimulatory domain, and a CD3ζ intracellular signaling domain. In this embodiment, the CAR may further comprise a signal peptide from B2M, CD8α, IgG1 or GM-CSFRα.

The present disclosure further provides a nucleic acid molecule encoding a chimeric antigen receptor targeting ROR1 as defined above, and a vector comprising the nucleic acid molecule.

As used herein, the term "vector" is an intermediary nucleic acid molecule used to transfer (exogenous) genetic material into a host cell, and in the host cell the nucleic acid molecule can be, for example, replicated and/or expressed. The vector generally includes targeting vectors and expression vectors. The "targeting vector" is a medium that delivers an isolated nucleic acid to the interior of a cell by, for example, homologous recombination or by using a hybridization recombinase specifically targeting a sequence at a site. The "expression vector" is a vector used for transcription of heterologous nucleic acid sequences (for example, those sequences encoding the chimeric antigen receptor polypeptides of the present disclosure) in suitable host cells and the translation of their mRNAs. Suitable vectors that can be used in the present disclosure are known in the art, and many are commercially available. In an embodiment, the vector of the present disclosure includes, but is not limited to, plasmid, virus (e.g., retrovirus, lentivirus, adenovirus, vaccinia virus, Rous sarcoma virus (RSV), polyoma virus, and adeno-associated virus (AAV), etc.), phage, phagemid, cosmid, and artificial chromosome (including BAC and YAC). The vector itself is usually a nucleic acid molecule, and usually is a DNA sequence comprising an insert (transgene) and a larger sequence as "backbone" of the vector. Engineered vector typically also comprises an origin of autonomous replication in the host cell (if stable expression of polynucleotide is desired), a selectable marker, and a restriction enzyme cleavage site (e.g., a multiple cloning site, MCS). The vectors may additionally comprise elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an in vitro transcription vector.

### Engineered immune cells

In an aspect, the present disclosure further provides an engineered immune cell expressing the recombinant receptor of the present disclosure, such as T cell fusion protein, T cell antigen coupler, recombinant TCR receptor or chimeric antigen receptor.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, an NK cell, and/or an NKT cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell may be any T cell, such as in vitro cultured T cell, for example, primary T cell, or T cell from in vitro cultured T cell line, e.g., Jurkat, SupT1, etc., or T cell obtained from a subject. Examples of subject include humans, dogs, cats, mice, rats, and transgenic species thereof. The T cell may be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be at any stage of development including, but not limited to, a CD4+/CD8+ T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell. In a preferred embodiment, the immune cell is a human T cell. The T cell may be isolated from the blood of a subject using a variety of techniques known to those of skill in the art, such as Ficoll.

The nucleic acid sequence encoding the chimeric antigen receptor can be introduced into an immune cell using conventional methods known in the art (e.g., by transduction, transfection, transformation). "Transfection" is a process of introducing a nucleic acid molecule or polynucleotide (including a vector) into a target cell. An example is RNA transfection, i.e., the process of introducing RNA (e.g., in vitro transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation may be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation. After the nucleic acid or vector is introduced into the immune cells, those skilled in the art may amplify and activate the obtained immune cells by conventional techniques.

In an embodiment, in order to reduce the risk of graft-versus-host disease, the engineered immune cell further comprises suppressed or silenced expression of at least one gene selected from the group consisting of: CD52, GR, dCK, TCR/CD3 genes (e.g., TRAC, TRBC, CD3γ, CD3δ, CD3ε, CD3ζ), MHC related genes (HLA-A, HLA-B, HLA-C, B2M, HLA-DPA, HLA-DQ, HLA-DRA, TAP1, TAP2, LMP2, LMP7, RFX5, RFXAP, RFXANK, CIITA) and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2 and GUCY1B3. Preferably, the engineered immune cell further comprises suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, CTLA4, more preferably TRAC, TRBC, HLA-A, HLA-B, HLA-C, B2M, RFX5, RFXAP, RFXANK, CIITA.

Methods of inhibiting gene expression or silencing genes are well known to those skilled in the art. For example, antisense RNA, RNA decoys, RNA aptamers, siRNA, shRNA/miRNA, trans dominant negative protein (TNP), chimeric/antibody conjugates, chemokine ligands, anti-infective cellular proteins, intracellular antibodies (sFv), nucleoside analogs (NRTI), non-nucleoside analogs (NNRTI), integrase inhibitors (oligonucleotides, dinucleotides, and chemical agents), and protease inhibitors may be used to inhibit the expression of genes. Alternatively, genes can also be silenced by DNA breakage mediated by for example meganucleases, zinc finger nucleases, TALE nucleases or Cas enzymes in CRISPR systems.

In an embodiment, the engineered immune cells further comprise a second chimeric antigen receptor targeting an tumor antigen different from ROR1. The tumor antigen different from ROR1 targeted by the second chimeric antigen receptor may be selected from, for example, the group consisting of : BCMA, CD4, CD5, CD7, CD8, CD14, CD15, CD19, CD20, CD21, CD22, CD23, CD25, CD33, CD37, CD38, CD40, CD40L, CD46, CD52, CD54, CD80, CD126, CD138, B7, MUC-1, HM1.24, angiogenic factor, VEGF, PIGF, ED-Bfibronectin, CD66a-d, IL-2, T101, TAC, IL-6, DR4, DR5, tEGFR, Her2, L1-CAM, mesothelin, CEA, hepatitis B surface antigen, antifolate receptor, CD24, CD30, CD44, EGFR, EGP-2, EGP-4, EPHa2, ErbB2, ErbB3, ErbB4, ErbB dimer, EGFR vIII, FBP, FCRL5, FCRH5, fetal acetylcholine receptor, GD2, GD3, GPRC5D, HMW-MAA, IL-22R-α, IL-13R-α2, κ light chain, Lewis Y, L1-CAM, MAGE-A1, MAGE-A3, MAGE-A6, PRAME, survivin, EGP2, EGP40, TAG72, B7-H6, IL-13Ra2, CA9, CD171, G250/CAIX, HLA-A1, HLA-A2, NY-ESO-1, PSCA, folate receptor-a, CD44v6, CD44v7/8, avb6 integrin, 8H9, NCAM, VEGF receptor, 5T4, fetal AchR, MUC1, MUC16, NY-ESO-1, MART-1, gplOO, carcinoembryonic antigen, VEGF-R2, CEA, prostate specific antigen, PSMA, Her2/neu, estrogen receptor, progesterone receptor, ephrin B2, CD123, c-Met, GD-2, O-acetylated GD2 (OGD2), CE7, WT-1, cyclin A2, CCL-1, hTERT, MDM2, CYP1B, WT1, survivin, AFP, p53, D1, CS-1, BAFF-R, TACI, CD56, TIM-3, CD123, L1-cell adhesion molecule, MAGE-A1, MAGEA3, CCNA1 and/or pathogen-specific antigens, biotinylated molecules, molecules expressed by HIV, HCV, HBV and/or other pathogens.

In an embodiment, a plurality of immune cells is provided, each immune cell engineered to express one or more chimeric antigen receptors. For example, in some embodiments, an immune cell is engineered to express a chimeric antigen receptor that binds and/or targets ROR1 (e.g., a CAR comprising the anti-ROR1 antibody of the present disclosure), and another cell is engineered to express a chimeric antigen receptor that binds and/or targets antigens different from ROR1. In an embodiment, immune cells may also express a multispecific chimeric antigen receptor that targets one or more antigens, including ROR1. For example, such a multispecific chimeric antigen receptor may comprise a multispecific antibody targeting ROR1, or comprise both the anti-ROR1 antibody of the present disclosure and one or more antibodies targeting antigen(s) different from ROR1. In such embodiments, the plurality of engineered immune cells may be administered together or separately. In an embodiment, the plurality of immune cells may be in the same composition or in different compositions. Exemplary compositions of cells include those described in the following sections of this application.

### Antibody conjugate

In an aspect, the present disclosure provides an antibody conjugate comprising the anti-ROR1 antibody as defined herein and a second functional structure, wherein the second functional structure is selected from the group consisting of an Fc, a radioisotope, a structure moiety for extending half-life, a detectable marker and a drug.

In an embodiment, the present disclosure provides an antibody conjugate comprising the anti-ROR1 antibody as defined in the present disclosure and Fc. As used herein, the term "Fc" is used to define the C-terminal region of an immunoglobulin heavy chain, and includes natural Fc and variant Fc. "Natural Fc" refers to a molecule or sequence comprising a non-antigen-binding fragment, whether monomeric or multimeric, produced by digestion of an intact antibody. The source of immunoglobulin from which natural Fc is produced is preferably of human origin. Natural Fc fragments are composed of monomeric polypeptides that can be linked as dimers or multimers through covalent linkages (e.g., disulfide bonds) and non-covalent linkages. Depending on the class (e.g., IgG, IgA, IgE, IgD, IgM) or subtype (e.g., IgG1, IgG2, IgG3, IgA1, IgGA2), natural Fc molecules have 1-4 intermolecular disulfide bonds between monomeric subunits. An example of a natural Fc is a disulfide-linked dimer produced by papain digestion of IgG (see Ellison et al. (1982), Nucleic Acids Res. 10:4071-9). The term "natural Fc" as used herein generally refers to monomeric, dimeric and multimeric forms. A "variant Fc" refers to an amino acid sequence that differs from that of a "natural" or "wild-type" Fc by virtue of at least one "amino acid modification" as defined herein, also referred to as an "Fc variant". Thus, "Fc" also includes single-chain Fc (scFc), i.e., a single-chain Fc consisting of two Fc monomers linked by a polypeptide linker, which is capable of naturally folding into a functional dimeric Fc region. In an embodiment, the Fc is preferably the Fc of a human immunoglobulin, more preferably the Fc of a human IgG1.

In an embodiment, the present disclosure provides an antibody conjugate comprising the anti-ROR1 antibody as defined in the present disclosure and a radioactive isotope. Examples of radioisotopes useful in the present disclosure include, but are not limited to, At²¹¹, I¹³¹, I¹²⁵, Y⁹⁰, Re¹⁸⁶, Re¹⁸⁸, Sm¹⁵³, Bi²¹², P³², Pb²¹², ^{99m}Tc, ¹²³I, ¹⁸F, and ⁶⁸Ga.

In an embodiment, the present disclosure provides an antibody conjugate comprising the anti-ROR1 antibody as defined in the present disclosure and a structure moiety for extending half-life selected from the group consisting of an albumin-binding structure of, a transferrin-binding structure, a polyethylene glycol molecule, a recombinant polyethylene glycol molecule, a human serum albumin, a fragment of human serum albumin, and a polypeptide binding to human serum albumin (including antibody).

In an embodiment, the present disclosure provides an antibody conjugate comprising the anti-ROR1 antibody as defined in the present disclosure and a detectable marker. The term "detectable marker" means herein a compound that produces a detectable signal. For example, the detectable marker may be an MRI contrast agent, a scintigraphy contrast agent, an X-ray imaging contrast agent, an ultrasound contrast agent, an optical imaging contrast agent. Examples of detectable markers include fluorophores (e.g., fluorescein, Alexa, or cyanine), chemiluminescent compounds (e.g., luminol), bioluminescent compounds (e.g., luciferase or alkaline phosphatase), enzymes (e.g., horseradish peroxidase, glucose-6-phosphatase, β-galactosidase), antibiotics (e.g., kanamycin, ampicillin, chloramphenicol, tetracycline, etc.) resistance genes, and contrast agents (e.g., nanoparticles or gadolinium). Those skilled in the art can select an appropriate detectable marker according to the detection system used.

In an embodiment, the present disclosure provides an antibody conjugate comprising the anti-ROR1 antibody as defined in the present disclosure and a drug conjugated to the anti-ROR1 antibody, such as a cytotoxin or an immunomodulator (i.e., an antibody-drug conjugate). Usually, the drug is covalently linked to the antibody, usually by a linker. In an embodiment, the drug is a cytotoxin. In another embodiment, the drug is an immunomodulator. Examples of cytotoxins include, but are not limited to, methotrexate, aminopterin, 6-mercaptopurine, 6-thioguanine, cytarabine, 5-fluorouracil, dacarbazine, nitrogen mustard, thiotepa, chlorambucil, melphalan, carmustine (BSNU), lomustine (CCNU), 1-methylnitrosourea, cyclophosphamide, nitrogen mustard, busulfan, dibromomannitol, streptozocin, mitomycin, cis-dichlorodiamine platinum (II) (DDP), cisplatin, carboplatin, zorubicin, doxorubicin, detorubicin, carminomicin, idarubicin, epirubicin, mitoxantrone, actinomycin D, bleomycin, calicheamicin, mithramycin, anthramycin (AMC), vincristine, vinblastine, paclitaxel, ricin, pseudomonas exotoxin, gemcitabine, cytochalasin B, gramicidin D, ethidium bromide, emetine, etoposide, teniposide, colchicine, mitoxantrone, 1-dehydrotestosterone, glucocorticoid, procaine, tetracaine, lidocaine, propranolol, puromycin, procarbazine, hydroxyurea, asparaginase, corticosteroids, mitotane (O,P'-(DDD)), interferon, and a combination thereof. Examples of immunomodulators include, but are not limited to, ganciclovir, etanercept, tacrolimus, sirolimus, voclosporin, cyclosporine, rapamycin, cyclophosphamide, azathioprine, mycophenolate mofetil, methotrexate, glucocorticoid and analogs thereof, cytokines, stem cell growth factors, lymphotoxins, tumor necrosis factor (TNF), hematopoietic factors, interleukins (e.g., IL-1, IL-2, IL-3, IL-6, IL-10, IL-12, IL-18 and IL-21), colony-stimulating factors (e.g., G-CSF and GM-CSF), interferons (e.g., interferon-α, interferon-beta and interferon-gamma), stem cell growth factor designated "S1 factor", erythropoietin and thrombopoietin, or a combination thereof.

### Kits and pharmaceutical compositions

In another aspect, the present disclosure further provides a detection kit comprising the humanized antibody, the multispecific antibody, the antibody conjugate, the engineered immune cell or the chimeric antigen receptor described in the present disclosure.

In another aspect, the present disclosure further provides a pharmaceutical composition comprising the humanized antibody, the chimeric antigen receptor, the multispecific antibody, the engineered immune cell or the antibody conjugate described in the present disclosure, and one or more pharmaceutically acceptable excipients.

As used herein, the term "pharmaceutically acceptable excipient" refers to a carrier and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, cosolvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the administration is parenterally accomplished. Parenteral delivery methods comprise topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also may be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210, bismuth 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic/preventive/diagnostic use

In another aspect, the present disclosure further provides a method for treating and/or preventing and/or diagnosing diseases associated with ROR1 expression, comprising administering to a subject the humanized antibody, the chimeric antigen receptor, the multispecific antibody, the antibody conjugate, the engineered immune cell or the pharmaceutical composition as described above.

In an embodiment, diseases associated with ROR1 expression include but are not limited to B-cell leukemia, lymphoma, B-cell chronic lymphocytic leukemia (CLL), non-Hodgkin's lymphoma (NHL), acute myeloid leukemia (AML), acute lymphoblastic leukemia (ALL), Burkitt lymphoma, mantle cell lymphoma (MCL), non-small cell lung cancer (NSCLC), neuroblastoma, kidney cancer, colon cancer, colorectal cancer, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, uterine cancer , adrenal cancer, and head and neck cancer.

The present disclosure will be described in detail below with reference to the accompanying drawings and examples. It should be noted that those skilled in the art should understand that the drawings and the embodiments of the present disclosure are only for the purpose of illustration, and shall not constitute any limitation to the present disclosure. In the case of no contradiction, the embodiments in the present application and the features in the embodiments can be combined with each other.

### Examples

### Example 1. Preparation of anti-ROR1 antibodies

The sequence encoding the extracellular region of ROR1 protein was cloned into the pCP vector with His tag, and CHO cells were transiently infected with the correctly sequenced plasmids. The cell culture was harvested after 8-10 days of culture, and the protein was purified by affinity chromatography to obtain the extracellular region of human ROR1 protein as an immunogen.

6-8 week old female Balb/c mice were used for primary and booster immunization, and ELISA and FACS were used to detect the antibody titer and specificity of the protein immunogen in serum. The mice were given a final booster immunization after comprehensive evaluation. After 3-4 days, the mice were sacrificed, the splenocytes were collected, mixed with mouse myeloma cells (SP2/0) according to a certain ratio, and the proliferated hybridoma cells were identified by FACS and ELISA to screen positive cell lines. After multiple rounds of subcloning identification, hybridoma cells that can stably secrete anti-ROR1 antibodies were finally obtained. Five anti-ROR1 antibodies were obtained after purification of the antibodies in the supernatant of the hybridoma cells.

Trypsin was used for enzymolysis of antibodies, and high-quality first-order and second-order spectra were obtained through DNA data collection, and the spectra were de novo analyzed with peaks software to obtain preliminary amino acid sequence results. Various proteases were used for enzymolysis of antibodies, and after MSE data collection, intact and fragmented information for all peptide peaks and for each valence state of each peptide could be obtained. The more comprehensive mass spectrometry data obtained were matched with the preliminary amino acid sequences, and the amino acid sequences of the five anti-ROR1 antibodies finally obtained are shown in Table 1 below.

**Table 1. Sequences of anti-ROR1 Antibodies**

| | ROR1-1 | ROR1-2 | ROR1-3 | ROR1-4 | ROR1-5 |
|---|---|---|---|---|---|
| CDR-L1 | SEQ ID NO: 1 | SEQ ID NO: 1 | SEQ ID NO: 10 | SEQ ID NO: 16 | SEQ ID NO: 1 |
| CDR-L2 | SEQ ID NO: 2 | SEQ ID NO: 2 | SEQ ID NO: 11 | SEQ ID NO: 17 | SEQ ID NO: 22 |
| CDR-L3 | SEQ ID NO: 3 | SEQ ID NO: 7 | SEQ ID NO: 12 | SEQ ID NO: 18 | SEQ ID NO: 7 |
| CDR-H1 | SEQ ID NO: 4 | SEQ ID NO: 4 | SEQ ID NO: 13 | SEQ ID NO: 19 | SEQ ID NO: 4 |
| CDR-H2 | SEQ ID NO: 5 | SEQ ID NO: 8 | SEQ ID NO: 14 | SEQ ID NO: 20 | SEQ ID NO: 5 |
| CDR-H3 | SEQ ID NO: 6 | SEQ ID NO: 9 | SEQ ID NO: 15 | SEQ ID NO: 21 | SEQ ID NO: 23 |
| VL | SEQ ID NO: 24 | SEQ ID NO: 27 | SEQ ID NO: 30 | SEQ ID NO: 33 | SEQ ID NO: 36 |
| VH | SEQ ID NO: 25 | SEQ ID NO: 28 | SEQ ID NO: 31 | SEQ ID NO: 34 | SEQ ID NO: 37 |
| scFv | SEQ ID NO: 26 | SEQ ID NO: 29 | SEQ ID NO: 32 | SEQ ID NO: 35 | SEQ ID NO: 38 |

### Example 2. Preparation of CAR-T cells comprising anti-ROR1 murine antibodies

Sequences encoding the following proteins were synthesized and cloned into pLVX vector (Public Protein/Plasmid Library (PPL), Cat. No.: PPL00157-4a): CD8α signal peptide (SEQ ID NO: 58), anti-ROR1 antibody, CD8α hinge region (SEQ ID NO: 60), CD8α transmembrane region (SEQ ID NO: 44), 4-1BB intracellular region (SEQ ID NO: 50) and CD3ζ intracellular signaling domain (SEQ ID NO: 52), and the correct insertion of the target sequence was confirmed by sequencing. The amino acid sequence of the anti-ROR1 scFv comprised in the ROR1-1 CAR is set forth in SEQ ID NO: 26; the amino acid sequence of the anti-ROR1 scFv comprised in the ROR1-2 CAR is set forth in SEQ ID NO: 29; the amino acid sequence of the anti-ROR1 scFv comprised in the ROR1-3 CAR is set forth in SEQ ID NO: 32; the amino acid sequence of the anti-ROR1 scFv comprised in the ROR1-4 CAR is set forth in SEQ ID NO: 35; the amino acid sequence of the anti-ROR1 scFv comprised in the ROR1-5 CAR is set forth in SEQ ID NO: 38.

Three ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector: viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask containing 293T cells. Viruses were collected at 24 hours and 48 hours, pooled, and ultracentrifuged (25000g, 4°C, 2.5 hours) to obtain concentrated lentivirus.

T cells were activated with DynaBeads CD3/CD28 CTS^{™} (Gibco, Cat. No. 40203D), and were further cultured for 1 day at 37°C and 5% CO₂. Then, the concentrated lentivirus was added, and after 3 days of continuous culture, CAR T cells expressing different anti-ROR1 murine scFv were obtained. Unmodified wild-type T cells were used as negative controls (NT).

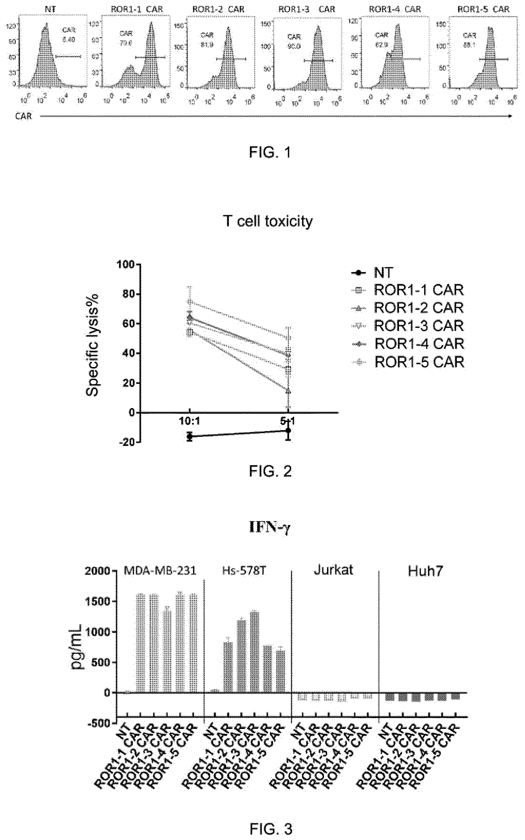

The scFv expression level of the ROR1-1 CAR-T cells, ROR1-2 CAR-T cells, ROR1-3 CAR-T cells, ROR1-4 CAR-T cells and ROR1-5 CAR-T cells was detected by flow cytometry using Biotin-SP (long spacer) AffiniPure Goat AntiMouse IgG, F(ab')₂ Fragment Specific (min X Hu, Bov, Hrs Sr Prot) (jackson immunoresearch, Cat. No. 115-065-072) as the primary antibody, APC Streptavidin (BD Pharmingen, Cat. No. 554067) or PE Streptavidin (BD Pharmingen, Cat. No. 554061) as the secondary antibody, and the results are shown in FIG. 1.

FIG. 1 shows that the anti-ROR1 scFv are effectively expressed on the CAR-T cells comprising different anti-ROR1 murine scFvs prepared by the present disclosure.

### Example 3. Detection of the killing effect of CAR-T cells expressing anti-ROR1 murine scFv on target cells and release of cytokines

### 3.1 Detection of the killing effect of CAR-T cells on target cells

MDA-MB-231 target cells carrying the fluorescein gene were plated in a 96-well plate at a concentration of 1×10⁴ cells/well, and then, NT cells and CAR-T cells expressing anti-ROR1 murine scFvs were plated into the 96-well plate at an effector-to-target ratio (i.e., the ratio of effector T cells to target cells) of 10:1 or 5:1 for co-culture. After 16-18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in FIG. 2.

FIG. 2 shows that compared to NT, the CAR-T cells expressing anti-ROR1 murine scFvs prepared by the present disclosure have significant specific killing effect on target cells under different effector-to-target ratios.

### 3.2 Detection of cytokine release level of CAR-T cells

Target cells (MDA-MB-231 cells, Hs-578T cells) or non-target cells (Jurkat cells, Huh7 cells) were plated in a 96-well plate at a concentration of 1×10⁵ cells/well, and then CAR-T cells expressing anti-ROR1 murine scFvs or NT cells were added at a ratio of 1:1 for co-culture, and the cell co-culture supernatant was collected after 18-24 hours.

A 96-well plate was coated with Purified anti-human IFN-γ Antibody (Biolegend, Cat. No. 506502) as capture antibody and incubated overnight at 4°C, and then the antibody solution was removed. 250 µL of PBST (1XPBS containing 0.1% Tween) solution containing 2% BSA (sigma, Cat. No. V900933-1kg) was added, and incubated at 37°C for 2 hours. The plate was then washed 3 times with 250 µL PBST (1XPBS containing 0.1% Tween). 50 µL of cell co-culture supernatant or standard per well was added and incubated at 37 °C for 1 h, then the plate was washed 3 times with 250 µL of PBST (1XPBS containing 0.1% Tween). Then 50 µL Anti-Interferon gamma antibody [MD-1] (Biotin) (abcam, Cat. No. ab25017) as detection antibody was added to each well, incubated at 37°C for 1 hour, and the plate was washed 3 times with 250 µL PBST (1XPBS containing 0.1% Tween). Then HRP Streptavidin (Biolegend, Cat. No. 405210) was added, incubated at 37°C for 30 minutes, and the supernatant was discarded. 250 µL PBST (1XPBS containing 0.1% Tween) was added for washing 5 times. 50 µL of TMB substrate solution was added to each well. Reactions were allowed to occur at room temperature in the dark for 30 minutes, after which 50 µL of 1 mol/L H₂SO₄ was added to each well to stop the reaction. Within 30 minutes of stopping the reaction, a microplate reader was used to detect the absorbance at 450 nm, and the content of cytokines was calculated according to the standard curve (drawn according to the reading value and concentration of the standard), the results are shown in FIG. 3.

It can be seen that the cells expressing anti-ROR1 murine scFvs prepared by the present disclosure did not release cytokine IFN-γ after co-culture with non-target cells Jurkat and Huh27, but released significantly increased IFN-γ after co-culture with target cells MDA-MB-231 cells or Hs-578T cells. This shows that the CAR-T cells expressing anti-ROR1 murine scFvs prepared by the present disclosure specifically kill target cells.

### Example 4. Humanization of anti-ROR1 Antibody

The murine antibody ROR1-4 was humanized with the following method: firstly, searching for the human antibody sequence with high similarity by using the IG BLAST database (https://www.ncbi.nlm.nih.gov/igblast/), then replacing the FR regions in the single-chain antibody with the corresponding human sequences; and then replacing individual amino acid residues according to the different physicochemical properties of the amino acid residues. Finally 7 humanized scFvs were obtained, the sequences of which are shown in Table 2.

**Table 2. Sequences of anti-ROR1 humanized antibodies.**

| | VL | VH | ScFv (aa) | ScFv (nt) |
|---|---|---|---|---|
| 6V7 | SEQ ID NO: 68 | SEQ ID NO: 69 | SEQ ID NO: 70 | SEQ ID NO: 71 |
| 6V8 | SEQ ID NO: 72 | SEQ ID NO: 73 | SEQ ID NO: 74 | SEQ ID NO: 75 |
| 6V9 | SEQ ID NO: 76 | SEQ ID NO: 77 | SEQ ID NO: 78 | SEQ ID NO: 79 |
| 6V10 | SEQ ID NO: 80 | SEQ ID NO: 81 | SEQ ID NO: 82 | SEQ ID NO: 83 |
| 6V11 | SEQ ID NO: 84 | SEQ ID NO: 85 | SEQ ID NO: 86 | SEQ ID NO: 87 |
| 6V12 | SEQ ID NO: 88 | SEQ ID NO: 89 | SEQ ID NO: 90 | SEQ ID NO: 91 |
| 6V13 | SEQ ID NO: 92 | SEQ ID NO: 93 | SEQ ID NO: 94 | SEQ ID NO: 95 |

### Example 5. Preparation of CAR-T cells comprising anti-ROR1 humanized antibodies

Sequences encoding the following proteins were synthesized and cloned into pLVX vector (Public Protein/Plasmid Library (PPL), Cat. No.: PPL00157-4a): CD8α signal peptide (SEQ ID NO: 58), anti-ROR1 humanized antibody (one of SEQ ID NOs: 70, 74, 78, 82, 86, 90, 94), IgG4 hinge region (SEQ ID NO: 64), CD8α transmembrane region (SEQ ID NO: 44), 4-1BB intracellular region (SEQ ID NO: 50) and CD3ζ intracellular signaling domain (SEQ ID NO: 52), and the correct insertion of the target sequence was confirmed by sequencing.

According to the method described in Example 2, the above-mentioned vector was packaged into lentivirus and used to infect activated T cells to obtain CAR-T cells comprising anti-ROR1 humanized antibodies.

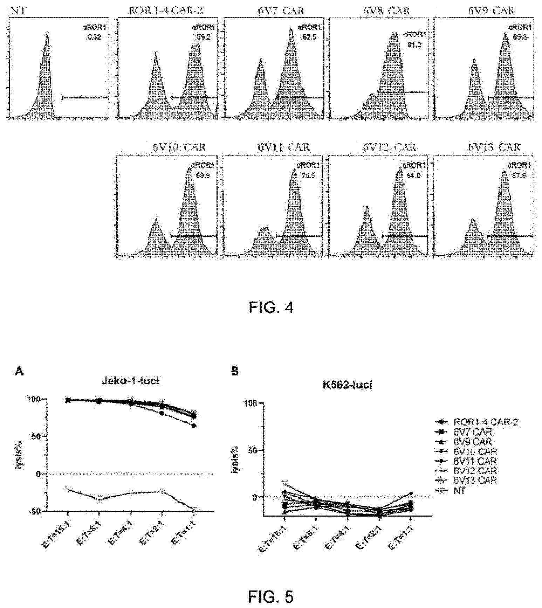

After culturing at 37°C and 5% CO₂ for 11 days, the above CAR-T cells, ROR1-4 CAR-T-2 cells (replacing the CD8α hinge region in the ROR1-4 CAR-T cells with the IgG4 hinge region) and Wild-type T cells (NT, used as negative control) were incubated with ROR1-hFc (Acrobio brand, Cat. No. RO1-H5250) protein for 45 minutes, and after washing, PE anti-human IgG Fc Antibody (Biolegend brand, Cat. No. 410708) was added to incubate for 30 minutes. The expression level of the anti-ROR1 single chain antibody on the CAR-T cells was detected by flow cytometry, and the results are shown in FIG. 4.

FIG. 4 shows that the anti-ROR1 humanized antibodies are effectively expressed in the CAR-T cells prepared by the present invention.

### Example 6. Detection of the killing effect on target cells, degranulation and cytokine release level of CAR-T cells expressing anti-ROR1 humanized scFvs

### 6.1 Detection of the killing effect of CAR-T cells on target cells

Target cells (Jeko-1-luci cells) or non-target cells (K562-luci cells) were plated into 96-well plates at a concentration of 1×10⁴ cells/well, and then, NT cells and CAR-T cells were plated into the 96-well plates at an effector-to-target ratio (i.e., the ratio of effector T cells to target cells) of 16:1, 8:1, 4:1, 2:1, and 1:1 for co-culture. After 16-18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in FIG. 5.

It can be seen that under different effector-to-target ratios, the CAR-T cells expressing anti-ROR1 humanized scFvs prepared by the present disclosure showed strong killing effect on target cells Jeko-1-luci cells, but weak killing effect on non-target cells K562-luci cells, indicating that CAR-T cells expressing anti-ROR1 humanized scFv only show specific killing effect on cells expressing ROR1.

### 6.2 Detection of degranulation of CAR-T cells

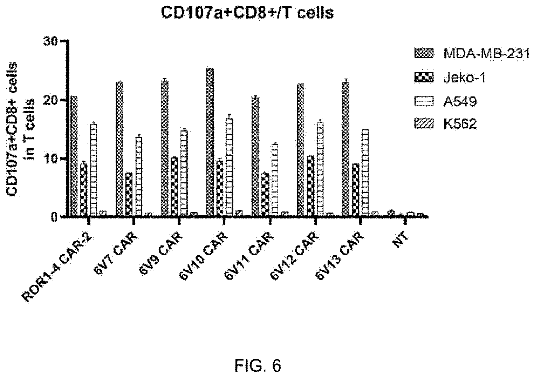

Target cells (MDA-MB-231 cells, Jeko-1 cells, A549 cells) and non-target cells (K562 cells) were plated in 96-well plates at a concentration of 1×10⁵ cells/well, CAR-T cells expressing anti-ROR1 humanized scFvs, ROR1-4 CAR-T-2 cells and NT cells (negative control) were added at a ratio of 1:1, respectively, then 10 µL of PE Mouse anti-human CD107a antibody (BD, Cat. No. 555801) was added to each well, and incubated at 37°C and 5% CO₂ in the dark. After 1 h, 20 µL of Golgi Stop (BD, Cat. No. 51-2092K2) was added to each well, and incubated at 37°C and 5% CO₂ in the dark for 2.5 h. Then, 10 µL of APC anti-human CD8 (BD, Cat. No. 555369) was added to each well, and incubated at 37°C and 5% CO₂ in the dark for 0.5 h. The cell samples in each well were detected by flow cytometry, and the ratio of CD107a+CD8+ cells to T cells was analyzed respectively, and the results are shown in FIG. 6 .

It can be seen that compared to NT cells, the CAR-T cells expressing ROR1 humanized scFvs prepared by the present invention showed significantly increased specific degranulation effects on three target cells MDA-MB-231 cells, Jeko-1 cells, and A549 cells, while no significantly increased degranulation was observed for non-target cells.

### 6.3 Detection of cytokine release level of CAR-T cells

Target cells (MDA-MB-231 cells, Jeko-1 cells, A549 cells) and non-target cells (K562 cells) were plated into 96-well plates at a concentration of 1×10⁵ cells/well, CAR-T cells expressing anti-ROR1 humanized scFvs and NT cells (negative control) were added at a ratio of 1:1, respectively, and the cell co-culture supernatant was collected after co-cultivation for 18-24 hours.

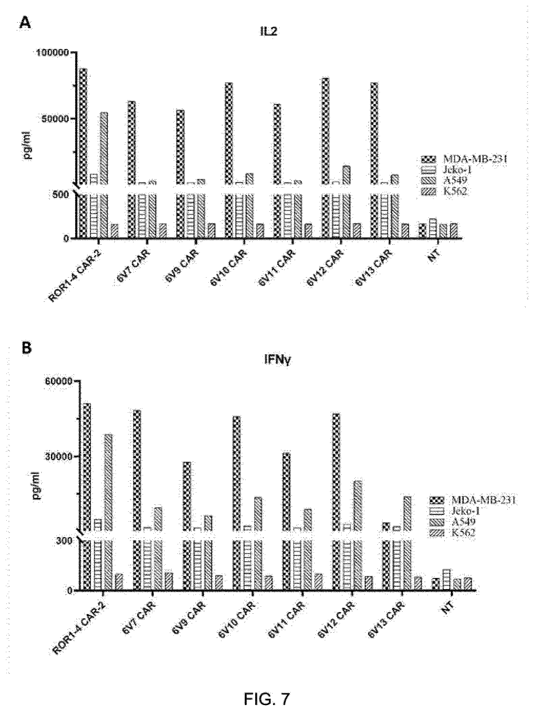

According to the manufacturer's recommendations, human IL-2 DuoSet ELISA Kit (R&D systems, Cat. No. DY202) and Human IFN-gamma DuoSet ELISA Kit (R&D systems, Cat. No. DY285) were used to detect the contents of IL2 and IFN-γ in the co-culture supernatant, and the results are shown in FIG. 7.

It can be seen that compared to NT cells, after the co-culture of CAR-T cells expressing ROR1 humanized scFvs of the present disclosure and three target cells, the release level of cytokines IL2 and IFN-γ was significantly increased, and the release of cytokines was specific.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. A ROR1-targeting antibody, comprising:
(1) CDR-L1 as set forth in SEQ ID NO: 1, CDR-L2 as set forth in SEQ ID NO: 2, CDR-L3 as set forth in SEQ ID NO: 3, CDR-H1 as set forth in SEQ ID NO: 4, CDR-H2 as set forth in SEQ ID NO: 5 and CDR-H3 as set forth in SEQ ID NO: 6;
(2) CDR-L1 as set forth in SEQ ID NO: 1, CDR-L2 as set forth in SEQ ID NO: 2, CDR-L3 as set forth in SEQ ID NO: 7, CDR-H1 as set forth in SEQ ID NO: 4, CDR-H2 as set forth in SEQ ID NO: 8 and CDR-H3 as set forth in SEQ ID NO: 9;
(3) CDR-L1 as set forth in SEQ ID NO: 10, CDR-L2 as set forth in SEQ ID NO: 11, CDR-L3 as set forth in SEQ ID NO: 12, CDR-H1 as set forth in SEQ ID NO: 13, CDR-H2 as set forth in SEQ ID NO: 14 and CDR-H3 as set forth in SEQ ID NO: 15;
(4) CDR-L1 as set forth in SEQ ID NO: 16, CDR-L2 as set forth in SEQ ID NO: 17, CDR-L3 as set forth in SEQ ID NO: 18, CDR-H1 as set forth in SEQ ID NO: 19, CDR-H2 as set forth in SEQ ID NO: 20 and CDR-H3 as set forth in SEQ ID NO: 21; or
(5) CDR-L1 as set forth in SEQ ID NO: 1, CDR-L2 as set forth in SEQ ID NO: 22, CDR-L3 as set forth in SEQ ID NO: 7, CDR-H1 as set forth in SEQ ID NO: 4, CDR-H2 as set forth in SEQ ID NO: 5 and CDR-H3 as set forth in SEQ ID NO: 23.

2. The antibody according to claim 1, **characterized in that** the antibody comprises a heavy chain variable region and a light chain variable region, the heavy chain variable region has at least 90% identity to an amino acid sequence selected from the group consisting of SEQ ID NOs: 25, 28, 31, 34, 37, 69, 73, 77, 81, 85, 89 and 93, or has one or several conservative modifications of amino acids compared to the amino acid sequence selected from the group consisting of SEQ ID NOs: 25, 28, 31, 34, 37, 69, 73, 77, 81, 85, 89 and 93; the light chain variable region has at least 90% identity to an amino acid sequence selected from the group consisting of SEQ ID NOs: 24, 27, 30, 33, 36, or has one or several conservative modifications of amino acids compared to the amino acid sequence selected from the group consisting of SEQ ID NOs: 24, 27, 30, 33, 36, 68, 72, 76, 80, 84, 88 and 92.

3. The antibody according to claim 1, **characterized in that** the amino acid sequence of the antibody has at least 90% identity to an amino acid sequence selected from the group consisting of SEQ ID NOs: 26, 29, 32, 35, 38, 70, 74, 78, 82, 86, 90 and 94, or has one or several conservative modifications of amino acids compared to the amino acid sequence selected from the group consisting of SEQ ID NOs: 26, 29, 32, 35, 38, 70, 74, 78, 82, 86, 90 and 94.

4. The antibody according to any one of claims 1-3, **characterized in that** the antibody is a murine antibody, a chimeric antibody, a humanized antibody or a human antibody.

5. The antibody according to any one of claims 1-4, **characterized in that** the antibody is selected from the group consisting of IgG, Fab, Fab', F(ab')₂, Fd, Fd', Fv, scFv, sdFv, linear antibody and diabody.

6. A nucleic acid molecule encoding the antibody according to any one of claims 1-5.

7. The nucleic acid molecule according to claim 6, **characterized in that** the nucleic acid molecule has at least 90%, at least 91%, at least 92%, at least 93%, at least 94%, at least 95%, at least 96%, at least 97%, at least 98%, at least 99%, 100% sequence identity to a nucleotide sequence selected from the group consisting of SEQ ID NOs: 39-43, 71, 75, 79, 83, 87, 91 and 95, and the antibody encoded thereby specifically binds to ROR1.

8. A multispecific antibody comprising the antibody according to any one of claims 1-5 and one or more second antibodies or antigen-binding portions thereof that specifically bind to antigens different from ROR1.

9. The multispecific antibody according to claim 8, **characterized in that** one or more second antibodies or antigen-binding portions thereof are selected from the group consisting of full-length antibody, Fab, Fab', (Fab')₂, Fv, scFv, scFv-scFv, minibody, diabody or sdAb.

10. A vector comprising a nucleic acid molecule encoding the antibody according to any one of claims 1-5 or the multispecific antibody according to claim 8 or 9.

11. A host cell expressing the antibody according to any one of claims 1-5 or the multispecific antibody according to claim 8 or 9.

12. A recombinant receptor comprising the antibody according to any one of claims 1-5 or the multispecific antibody according to claim 8 or 9, **characterized in that** the recombinant receptor is a T cell fusion protein, a T cell antigen coupler, a recombinant TCR receptor or a chimeric antigen receptor.

13. The recombinant receptor according to claim 12, further comprising a transmembrane domain and an intracellular signaling domain.

14. The recombinant receptor according to claim 13, **characterized in that** the transmembrane domain is a transmembrane domain of a protein selected from the group consisting of: a TCRα chain, a TCRβ chain, a TCRγ chain, a TCRδ chain, a CD3ζ subunit, a CD3ε subunit, a CD3γ subunit, a CD3δ subunit, CD45, CD4, CD5, CD8α, CD9, CD16, CD22, CD33, CD28, CD37, CD64, CD80, CD86, CD134, CD137, and CD154.

15. The recombinant receptor according to claim 13, **characterized in that** the intracellular signaling domain is an intracellular region of a protein selected from the group consisting of: FcRγ, FcRβ, CD3γ, CD3δ, CD3ε, CD3ζ, CD22, CD79a, CD79b, and CD66d.

16. The recombinant receptor according to claim 13, further comprising one or more co-stimulatory domains selected from the group consisting of co-stimulatory signaling domains of proteins: TLR1, TLR2, TLR3, TLR4, TLR5, TLR6, TLR7, TLR8, TLR9, TLR10, CARD11, CD2, ROR1, CD8, CD18, CD27, CD28, CD30, CD40, CD54, CD83, CD134, CD137, CD270, CD272, CD276, CD278, CD357, DAP10, LAT, NKG2C, SLP76, PD-1, LIGHT, TRIM, and ZAP70.

17. An engineered immune cell comprising the recombinant receptor according to any one of claims 12-16.

18. The engineered immune cell according to claim 17, **characterized in that** the engineered immune cell is selected from the group consisting of a T cell, an NK cell, an NKT cell, a macrophage, and a dendritic cell.

19. The engineered immune cell according to claim 18, further comprising a second chimeric antigen receptor targeting tumor antigens different from ROR1.

20. The engineered immune cell according to any one of claims 17-19, further comprising suppressed or silenced expression of at least one gene selected from the group consisting of: TRAC, TRBC, HLA-A, HLA-B, HLA-C, B2M, RFX5, RFXAP, RFXANK, CIITA, PD1, LAG3, TIM3, and CTLA4.

21. An antibody conjugate comprising the antibody according to any one of claims 1-5 or the multispecific antibody according to claim 8 or 9, and a second functional structure, **characterized in that** the second functional structure is selected from the group consisting of an Fc, a radioisotope, a structure moiety for extending half-life, a detectable marker and a drug.

22. The antibody conjugate according to claim 21, **characterized in that** the structure moiety for extending half-life is selected from the group consisting of an albumin-binding structure, a transferrin-binding structure, a polyethylene glycol molecule, a recombinant polyethylene glycol molecule, a human serum albumin, a fragment of human serum albumin, and a polypeptide binding to human serum albumin; the detectable marker is selected from the group consisting of a fluorophore, a chemiluminescent compound, a bioluminescent compound, an enzyme, an antibiotic resistance gene, and a contrast agent; and the drug is selected from the group consisting of a cytotoxin and an immunomodulator.

23. A detection kit comprising the antibody according to any one of claims 1-5, the multispecific antibody according to claim 8 or 9, the recombinant receptor according to any one of claims 12-16, the engineered immune cell according to any one of claims 17-20, or the antibody conjugate according to claim 21 or 22.

24. A pharmaceutical composition comprising the antibody according to any one of claims 1-5, the multispecific antibody according to claim 8 or 9, the recombinant receptor according to any one of claims 12-16, the engineered immune cell according to any one of claims 17-20, or the antibody conjugate according to claim 21 or 22, and one or more pharmaceutically acceptable excipients.

25. Use of the antibody according to any one of claims 1-5, the multispecific antibody according to claim 8 or 9, the recombinant receptor according to any one of claims 12-16, the engineered immune cell according to any one of claims 17-20, or the antibody conjugate according to claim 21 or 22, or the pharmaceutical composition according to claim 24 in the preparation of a medicine for treating and/or preventing and/or diagnosing a disease associated with ROR1 expression.

26. The use according claim 25, **characterized in that** the disease associated with ROR1 expression is selected from the group consisting of B-cell leukemia, lymphoma, B-cell chronic lymphocytic leukemia (CLL), non-Hodgkin's lymphoma (NHL), acute myeloid leukemia (AML), acute lymphoblastic leukemia (ALL), Burkitt lymphoma, mantle cell lymphoma (MCL), non-small cell lung cancer (NSCLC), neuroblastoma, kidney cancer, colon cancer, colorectal cancer, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, uterine cancer, adrenal cancer, and head and neck cancer.
